# EUROPEAN PATENT APPLICATION

(11) **EP 4 275 593 A1**
(43) Date of publication of application: **15.11.2023**
(21) Application number: 22172237.4
(22) Date of filing: 09.05.2022
(51) Int. Cl.: A61B 5/03, A61B 5/20, A61B 5/00, H01M 8/16, A61B 5/145

(54) **DISPOSABLE MEDICAL DEVICE ASSEMBLY WITH SENSOR**

(71) Applicant: Wellspect AB, 431 21 Mölndal (SE)
(72) Inventor: LOVMAR, Martin, 431 69 Mölndal (SE); CLAESSON, Henrik, 437 38 Lindome (SE); D'ESPINDULA, Gabriel, 418 71 Göteborg (SE); BERTHUEL, Marie, 38610 Gieres (FR); GUILBAUD, Lisa, 38660 Le Touvet (FR); HAMMOND, Jules, 38610 Gieres (FR); SOLAN, Sébastien, 38400 Saint Martin d Heres (FR); SERGEJEVS, Aleksandrs, 38610 Gieres (FR); THIERRY, Alban, 38100 Grenoble (FR); UTAS, Jan, 434 95 Kungsbacka (SE)
(74) Representative: Lind Edlund Kenamets Intellectual Property AB

(57) **Abstract**

A disposable medical device assembly for measuring a physiological parameter in a urinary or gastrointestinal tract of a mammal being is disclosed, comprising a probe, such as a catheter, for insertion into a bodily opening of the mammal being. The probe comprises at least one sensor, such as a pressure sensor. The medical device further comprises an energy source, in the form of a biofuel cell, connected to the at least one sensor.

## Description

### TECHNICAL FIELD OF THE INVENTION

The present invention relates to a disposable medical device assembly comprising a probe for insertion into a bodily opening, such as a catheter or a solid probe.

### BACKGROUND OF THE INVENTION

Probes, such as catheters, are known to be inserted into bodily openings of humans and other mammals. For example, urinary catheters are commonly used for insertion into the urethra, for drainage of urine. Similarly, solid probes and catheters are well known for insertion into the rectum, e.g. for anal irrigation.

Some catheters and other probes are intended for short time use, such as intermittent urinary catheters, intended to be inserted for a short period of time, such as a few minutes, and then be withdrawn again. Other catheters and probes are intended to be used for longer time periods, such as Foley catheters, intended to be inserted for hours, days or even weeks. Catheters and probes for long time use are often provided with retention elements, such as inflatable balloons, to maintain the catheter/probe in position for the extended period of use.

It is further known to add some diagnostic functionality to catheters and other probes, such as Foley catheters, e.g. to measure pressure and temperature. For example, US 5389217 discloses a catheter with oxygen sensing capability, US 5916153 and US 6434418 both disclose a pressure sensor associated with a Foley type catheter, and US 6602243 discloses a temperature sensor associated with a Foley type catheter. Further, US2013030262 discloses a Foley catheter with a pressure sensor.

Despite the benefits of incorporating sensors for measuring of physiological parameters in catheters and probes, there are also several drawbacks with such an approach. The catheters/probes incorporating such functionality becomes complicated and costly to produce. Further, the catheters/probes including such functionality include many different materials, including various types of metals, which make them difficult to handle, recover, sort, process and dispose of in an environmentally friendly way after use. Still further, these known catheters/probes are difficult to use, and the measurement data provided is often inaccurate or unreliable. Further, if a catheter/probe is to be reused, it must be properly cleaned, sterilized, inspected and recalibrated if the probe/catheter comprises one or more sensor(s). This is time consuming and costly.

There is therefore a need for catheters/probes which are capable of providing reliable physiological data in a more cost-efficient way, and with catheters/probes which are more suitable for environmentally friendly handling after use.

### SUMMARY OF THE INVENTION

It is therefore an object of the present invention to provide a medical device assembly which at least alleviates the above-discussed problems.

This object is obtained by means of a medical device assembly in accordance with the appended claims.

According to a first aspect of the invention there is provided a disposable medical device assembly for measuring a physiological parameter in a urinary or gastrointestinal tract of a mammal being, the assembly comprising:
a probe for insertion into a bodily opening of said mammal being, said probe comprising at least one sensor; and
an energy source connected to said at least one sensor;
wherein said energy source is a biofuel cell, and preferably a biodegradable and/or compostable biofuel cell. Preferably, the biofuel cell is also free of metal.

The present invention is based on the realization that it may be highly advantageous to measure and monitor one or more physiological parameters in the urinary and/or gastroinstestinal tract in a simple and convenient way, to be able to determine and/or foresee medical conditions or problems, and to evaluate the medical health of a user/patient.

Such measuring and analysis have heretofore been rather, tedious, complicated, and costly, involving complicated and expensive measurement equipment, handling, etc.

The present invention provides a relatively simple and cost-efficient probe, which may easily be inserted into the urethra, rectum, or the like, for measuring of one or more physiological parameters. Since the device assembly is disposable, it may then be discarded after use, thereby simplifying the procedure even further.

In the context of the present application, probe is to be understood as an elongate device capable of being introduced into a mammal body, e.g. into an natural or artificial body opening. The probe may be used for measuring or exploring within the body, but may also be used for drainage of fluid, insertion of fluid, etc.

The probe may be dedicated for the intended measurement but may alternatively be usable also for other purposes. For example, the probe may be formed as a closed, solid substrate, having the purpose of being insertable into the site where the measurement is intended to take place, such as at a position when inserted into the urethra or rectum of a human or other mammal. Alternatively, the probe may also be a catheter or the like, having a lumen allowing for transport of a fluid. A catheter is a tubular probe, having an internal lumen extending between a inlet/outlet opening arranged at one end and a discharge/drainage opening at an opposite end. The catheter may hereby also function as a drainage catheter for draining urine or the like, as an irrigation catheter/probe, for introduction of an irrigation liquid into a mammal cavity, e.g. for anal irrigation, or the like.

Thus, the probe may be a solid probe, or a probe with one or more internal cavities. The probe may also be provided with one or more lumens extending between a distal end a proximal end. At least one lumen may extend between a proximal, insertable opening, and a distal opening. In case of a urinary catheter, the lumen may extend between a drainage opening in the proximal, insertable end, and a discharge opening in the distal, non-insertable end.

When the probe is in the form of a catheter, such as a rectal or urinary catheter, the catheter may have a single lumen, and preferably be free of any retention element. Such a catheter is particularly suited for relatively short time use, such as for a duration of a few minutes or the like. However, the catheter may also have two or more lumens. Such a catheter may e.g. have an inflatable retention element, i.e. a balloon, which is inflatable to retain the catheter in place when inserted. Such a catheter may be used for longer periods of time, such as for a few hours, or even days or weeks. Such an indwelling catheter may e.g. be of the type commonly referred to as Foley catheter.

The probe/catheter may be provided with a coating on an outer surface, e.g. to reduce the friction during insertion. The coating may e.g. be a hydrophilic coating. The hydrophilic coating can be wetted prior to use, e.g. with water or saline, and thereby swells and obtains lower friction.

In case a hydrophilic coating is provided on the catheter/probe, the catheter probe can be activated and wetted immediately prior to use. It is also feasible to arrange a supply of wetting fluid in a package containing the catheter/probe. In such embodiments, the wetting fluid may be arranged in a separate container or compartment of the package, thereby allowing the wetting fluid to be released for activation of the hydrophilic coating before opening of the package. In other embodiments, the wetting fluid may be arranged in the package so that the hydrophilic coating is maintained in a wetted, activated state during storage, and thereby to be ready for immediate use without any initial preparation steps.

Other types of coatings may also be used, such as a coating of silicone. A coating may also be used to provide antibacterial properties to the probe, i.e. be an antibacterial coating. Other coatings than hydrophilic coatings may also be used to provide a low friction at the surface, such as a PTFE coating.

By "disposable" is in the context of the present application meant a product which can be disposed after a single and/or short time use, in an environmentally friendly way. The probe of the disposable medical device assembly is preferably made of a biodegradable material. Put differently, the materials used in the disposable product are preferably to a large extent, and preferably totally, biodegradable and/or compostable. Thus, the disposable medical device, or at least parts thereof, are preferably biobased, biodegradable and/or compostable, and most preferably at least the entire probe is biodegradable and compostable. By a biodegradable material is here meant an organic material which breaks down by microorganisms, such as bacteria and fungi, in a microbial process, and preferably under environmental exposure, e.g. making the material compostable. A biodegradable material may also be biodegradable in accordance with any of the standards ASTM D6868 and EN 13432, and may also be compostable in accordance with the standard ASTM D6400.

The biofuel cell may be made of or based on a bio sourced and/or biodegradable and/or bio compostable material. Such material(s) may be selected from the group comprising cellulose, natural carbon materials (graphite, black carbons), natural polymers (chitosan, alginate, starch, microcrystalline cellulose, carboxylate methyl cellulose, cellulose acetate, agar, xanthan gum, Polylactic acid, poly caprolactone). Biodegradable materials may further be selected among the bio sourced biodegradable materials, and further among paper, cardboard. The biofuel cell may be an enzymatic fuel cell or a microbiologic fuel cell, preferably made from vegetable fibers, for example paper-based. Vegetable fibers or paper-based biofuel cells are advantageous due to their low cost, the flexibility, allowing them to conform to various surfaces, and the low environmental impact. Examples of advantageous paper-based biofuel cells are those provided by the Company BeFC (French company). Advantageously, the paper-based bio fuel cell may be as disclosed in the published patent applications US 2021/0249676, WO 2021/170826 and WO 2021094593, said documents hereby incorporated in their entirety by reference. The biofuel cell is also preferably made of a biobased material.

At least one of the biofuel cell and the probe, and preferably both, are preferably made of a biobased material. Preferably, at least 50 wt% of the biofuel cell and/or probe are made of biobased material, and more preferably at least 70 wt%, and more preferably at least 90 wt%, and most preferably at least 99 wt%.

By biobased is here mean a material wholly or partly of biogenic origin, and in particular a material in accordance with one or more of the standards ISO 16620-1:2015, ISO 16620-2:2019, ISO 16620-3:2015, ISO 16620-4 and ISO 22526-2:2020.

The energy source can also be disposed of after use in a variety of ways, since it is preferably biodegradable, and preferably also compostable, non-toxic and free of metal. The energy source is also preferably free of any other materials which may be toxic or non-environmentally friendly.

The metal possibly needed for other, additional parts of the disposable medical device, such as connection lines, antennas, user interface(s), etc, can be very limited, thereby making the overall metallic content in the medical device insignificant and negligible, even for such embodiments.

The disposable medical device can be fully assembled and prepared for use already during manufacturing, such as being provided with the sensor already connected to the energy source, sterilized, etc. Thus, the medical device can be used almost immediately, or after only very few assembly or preparatory steps. After use, the medical device can easily be disposed, e.g. by being thrown in conventional and ubiquitous trash cans, dust-bins, garbage cans, etc., or by being thrown into separate containers for waste sorting dedicated to e.g. plastic, paper or compostable materials. This makes it easy for the user to get rid of the disposable medical device after use, and in an environmentally friendly manner, thereby reducing the environmental footprint.

The disposable medical device is primarily useable for humans, for introduction e.g. into the urethra and/or rectum. However, it is contemplated that the same or similar disposable medical devices may also be used on other mammals, and in particular pets and domestic cattle, such as dogs, cats, cows, horses, and the like.

The biofuel cell is preferably free of metal and is consequently an energy source well suited for a disposable product. The biofuel cell can e.g. in total, or to a great extent, be made of biodegradable and/or compostable materials.

Thus, there is no need to disassemble the product for disposal, such as by removing batteries and the like. On the contrary, the entire product can be disposed directly after use, and with full compliance of environmental regulations and with low environmental impact.

The disposable medical device is also cost-effective to produce and use. In particular, since the medical device is intended for one-time use, it can be produced and used in a very safe and hygienic way, and all steps normally required for re-usable products can be omitted, such as disassembling of parts, cleaning, re-sterilization, etc.

In particular, the biofuel cell may be an enzymatic fuel cell, and preferably be paper-based, or based on bio sourced polymers or biodegradable polymer. Such biofuel cells are advantageous due to their low cost, the flexibility, allowing them to conform to various surfaces, and the low environmental impact. The biofuel cell can also be sterilized, which makes it suitable for medical use.

In an embodiment, the biofuel cell is made of bio-polymers, enzymes or bacteria and carbons. The biofuel cell may comprise a microbial and/or enzymatic solution.

The biofuel cell can in such embodiments be made extremely thin, flexible and small, which makes it well adapted to be integrated in medical devices.

The biofuel cell may be arranged to use biological catalysts instead of chemical or expensive noble metal catalysts to convert natural substrates such as glucose and oxygen into electricity. The biofuel cell preferably uses biofuel enzyme cells to produce electrical energy from biological substances, such as oxygen and/or glucose, which are present in biological fluids, such as sweat, blood and urine.

The biofuel cell may be a single cathode cell (SC), where a cathode is positioned between an anode and a support. Alternatively, the biofuel cell may be realized as a single cathode air cell (SABC)) or a double cathode air cell (DABC). In a SABC realization, the support of the SC realization may be replaced with a support which is permeable to air and allows penetration of oxygen. In a DABC realization, the biofuel cell comprises two cathodes arranged on each side of an anode, and with permeable support layers arranged on the outside of each of the two cathodes. However, other realizations are also feasible, as is per se known in the art.

The anode(s) can e.g. be made of a solid agglomerate comprising a conductive material mixed with a first enzyme capable of catalyzing the oxidation of the biofuel. Similarly, the cathode(s) can e.g. be made of a solid agglomerate comprising a conductive material mixed with a second enzyme capable of catalyzing the reduction of oxidant.

Biofuel cells suitable for use for the disposable medical device are e.g. the biofuel cells disclosed in US 2021/0249676, WO 2021/170826 and WO 2021/094593, all of which are hereby incorporated in their entirety by reference.

The one or more sensor(s) is useable to determine one or more physiological parameters in relation to the user. The measured sensor data can be used to monitor physiological parameters, and thereto related health related conditions, over longer or shorter periods of time. The measured sensor data can also be used for diagnostic purposes, and also as an aid in various forms of treatments.

The at least one sensor is preferably at least one of a pressure sensor, a temperature sensor, a position sensor, an imaging sensor, an accelerometer, a gyroscope, a pH sensor and a flow sensor.

In one embodiment, the at least sensor comprises at least one pressure sensor, also referable to as a pressure transducer. The pressure sensor can e.g. be a digital or solid state pressure sensor, as is per se known in the art. Such pressure sensors may include, by way of example, any of a piezoelectric electric mechanism, an optical sensing mechanism, or a microelectricalmechanical (MEMS) mechanism.

The disposable medical device can also be provided with two or more sensors, either of different types, for measuring of different physiological parameters, or of the same type, e.g. for measuring of the same physiological parameters at different positions.

The medical device may also comprise more than one pressure sensor, such as two, three or even more pressure sensors. In such an embodiment, the pressure sensors may e.g. be located at different positions along the length of the probe, thereby making it possible to determine the pressure at various positions in the body of the user. For example, the at least one sensor may comprise a sensor arranged in the vicinity of an insertable tip of the probe, and at least one sensor arranged on the insertable part of the probe at a distance form said insertable tip.

In particular when the probe is arranged for insertion into the urethra of the mammal being, at least two sensors may be provided: one arranged in the vicinity of an insertable tip of the probe, for measurement of a pressure inside the bladder, and at least one sensor arranged on the insertable part of the probe at a distance form said insertable tip, for measurement of the pressure inside the urethra. Further, the at least one sensor for measurement of the pressure inside the urethra may comprise one sensor arranged to measure the pressure in the vicinity of the prostate, and one sensor arranged to measure the pressure inside the urethra at a distance from the prostate.

The probe may e.g. be a urodynamic catheter or cystometry catheter. A urodynamic catheter is a catheter useable to estimate changes that occur inside the bladder and/or the urethra during filling or voiding. The urodynamic system might also contain a rectal probe useable to estimate abdominal pressure changes during filling or voiding. Such a urodynamic catheter is much easier and faster to use than conventionally known urodynamic catheters, and can also be used and produced more cost-effectively. In particular, there is hereby no need to use the device as a water containing system, where the bladder is actively filled with water during testing, and also need to transfer the pressure from the measuring points to an external sensor using a catheter with water-filled lumens and the like. Instead, with the present invention, the sensors may be arranged on the disposable probe to enable direct measurement of the pressure.

Further, there is hereby no need for dedicated urodynamic instrument and the like, since the measurement results can easily be shown in a smartphone app, on a personal computer, or the like.

In one embodiment, a medical device provided with a probe and one or more pressure sensor(s) can be usable as a simple obstruction testing device. Such a device could be used for forming a coarse overview of a situation or be used by a user in preparation for a visit to the urologist. Such a medical device is e.g. well suited for GP (general practitioner) setting or self-testing.

Such a medical device can be used in the following way: First, the single-use package in which the device is arranged is opened. The sterile probe is then extracted from the package, and inserted into the bodily opening, such as the urethra. The probe is then maintained in the inserted position. When the user feels a natural urge to void the bladder this is allowed to happen over the probe. To this end, the probe preferably has a relatively narrow cross-section, allowing urine to flow between the walls of the urethra and the probe. The pressure is measured in the sensors of the probe. The measurement data can in one embodiment be directly provided to a display or the like of the device, or additionally, or alternatively, be communicated to an external unit, for presentation, storing and or analysis of the measurement data. For example, the measurement data may be presented on a small display connected to the probe, and e.g. arranged to be positioned on the body, such as on the penis, during use. The connection may be wired or wireless. The display may e.g. show bars or other indications of the pressure measured at the various sensor positions, such as at B (bladder), P (prostate) and U (urethra). The sensors may e.g. be positioned to measure the bladder pressure, with a sensor arranged at the tip, the prostate pressure, with a sensor arranged intermediately on the probe, at a position close to the prostate, and the urethral pressure, with a sensor arranged at the probe at a position distant from both the tip and the prostate. For the measurement, the probe may be inserted for a certain period of time, such as a between a few minutes and a few hours. After the measurement, the single-use device may be retracted and discarded. The device may be discarded without any special considerations, such as any need for battery recycling.

Such measurements may be used to determine a condition of underactive bladder, an enlarged prostate, or the like. For example, a high bladder pressure in combination with a low urine flow and/or a low urethral pressure may indicate an obstruction, likely due to an enlarged prostate, while a low bladder pressure in combination with low flow during micturition may suggest that the low flow is related to an underactive bladder.

A similar device and procedure may also be used to determine the condition of overactive bladder (OAB). Hereby, a cost-efficient and relatively simple device may be provided capable of evaluation and determination of an OAB condition.

In a rectal catheter or probe, a pressure sensor may also be used to determine when there is a need for the user to defecate. Similarly, a pressure sensor may be used to determine problems related to the rectal sphincter, such as problems where the rectal sphincter has difficulty to close.

A pressure sensor close to the insertable tip may also be used to determine the pressure exerted by an inflatable retention element, and may also be used to control the inflation to achieve an optimal inflated size.

A medical device with one or more pressure sensor(s) may also be used for monitoring, controlling and/or evaluation of training and rehabilitation exercises, such as pelvic floor muscle training.

In some embodiments, the medical device further includes one or more analyte sensors. Analyte sensors included in the scope of the disclosed technology include sensors for analytes of any clinical significance. For broad examples, such analytes may include any analyte selected from a group including pH, a gas, an electrolyte, a metabolic substrate, a metabolite, an enzyme, or a hormone. By way of particular examples, such analyte sensor may be able to sense any of a metabolic substrate or a metabolite, the analytes may include glucose or lactic acid. By way of example of a hormone, the analyte may include cortisol.

At least one sensor may also be provided on the probe to determine presence of one or more substances in a bodily fluid, such as in urine. The sensors may be arranged to determine the presence in a qualitative way only, i.e. to determine whether there is a presence or not, or in a more quantitative way, i.e. to estimate also the quantity of the substance. For urine, the sensor(s) may be arranged to determine presence of one or more of the substances conventionally identifiable with so called urine test strips, such as one or more of proteins, glucose, hemoglobin, bilirubin, urobilinogen, acetone, nitrite and leucocytes. Similarly, sensor(s) may be provided to determine pH of the fluid, to determine a specific gravity and/or to test for infection by different pathogens.

Similarly, sensors may be provided to determine the presence of one or more substances in feces, such as the presence of fecal occult blood (FOB).

In some embodiments, the medical device includes a temperature sensor to monitor a body temperature of the patient/user. Temperature sensors appropriate for the probe may be of any conventional type, including by way of example, a thermistor, a thermocouple, or an optical temperature sensor.

In some embodiments, the medical device further includes one or more electrodes arranged as electrical activity sensors. Such electrical activity sensors may deliver physiologic data such as electromyography (EMG) or electrogastrogram (EGG).

In some embodiments, the medical device further includes a light source and a light sensor, the sensor configured to capture light emitted from the light source. In some embodiments, by way of example, the light source and the light sensor may be configured to operate as a pulse oximeter, the light sensor being able to deliver a signal that can be transduced into a pulse rate. In another example, the light source and the light sensor may be configured to operate as an analyte sensor.

The disposable medical device assembly may further comprise an interface arranged to transfer or transmit measurement data from said sensor to an external receiver. In one embodiment, the interface comprises a connector arranged to connect to a wire. In another embodiment, the interface is a wireless interface, and comprises a transmitter for wireless communication. The wired or wireless connection may be connected to an external device, such as an external personal computer, smartphone, tablet, or the like. Alternatively, the interface may in itself comprise a user interface device, such as a display and/or a speaker.

The external device and/or the interface device may be arranged to communicate data related to the measurement data to a user. The communication may e.g. be in the form of a presentation of the actual measured data, presentation of the measured data in aggregated form, and/or presentation of the measured data in the form of whether the measured values are above or below one or more threshold values.

The medical device may further be used to send measurement data of one or more physiological parameters to an external storage, such as a memory in the external device, enabling the data to be followed over time. Hereby, it becomes possible to detect trends in the data, and also to detect deviations from a normal condition. Such stored data may also be useable for evaluation of therapy compliance, follow-up during training and rehabilitation, trouble-shooting, etc. It also makes it possible for a user to use the stored data as a diary or register, to see e.g. when release of urine has occurred, the volume of the released urine, the flow rate, etc.

The disposable medical device assembly may further comprise an additional second probe, wherein the second probe also comprises at least one sensor. Hereby, it is e.g. possible to use one of the probes for insertion into the urethra, and use one probe for insertion into the rectum. The measurements may hereby be correlated to each other, and one of the measurements may e.g. be used as a reference for the other measurement.

The biofuel cell may be arranged on or in the probe, thereby forming an integrated part of the probe. The biofuel cell may be arranged on an insertable part of the probe, or alternatively on a non-insertable part. Alternatively, the biofuel cell may be arranged separated from the probe, and may e.g. be connected to the probe via a conducting wire.

In one embodiment, the biofuel cell is arranged at a distance from said at least one sensor, and wherein a conductor is provided in the probe forming a conductive path between the biofuel cell and the sensor(s).

A conductor connecting the biofuel cell with the one or more sensors may e.g. be realized as a ribbon cable. However, other forms of wired connections are also useable to enable transfer of electricity from the biofuel cell and electrical signals or data signals from the sensors.

The conductor/wire may be arranged on an external or internal surface of the probe, but may alternatively also be arranged enclosed within the probe.

The sensor(s) may be arranged on an outer surface of the probe, and may e.g. be affixed to the probe by adhesive or the like. However, additionally or alternatively, sensor(s) may be integrated in the probe, and/or be arranged inside the probe, such as in an internal lumen of the probe.

The probe may have an essentially circular cross-sectional shape. However, other shapes are also feasible, such as an oval shape and the like. In other embodiments, in particular where there is no lumen inside the probe, the probe may also have a relatively flat cross-sectional shape, and may e.g. be formed as a rod, a strip, or the like.

The probe is preferably made of a relatively flexible material, making the probe somewhat bendable. In particular a probe for insertion into the urethra may have properties similar to a conventional urinary catheter. A probe for insertion into the rectum may have similar properties, but may alternatively be larger and somewhat more stiff and rigid.

### BRIEF DESCRIPTION OF THE DRAWINGS

The present invention will be described in more detail with reference to the appended drawings, showing currently preferred embodiments of the invention. The drawings illustrate:
Fig. 1 is a sideview of an embodiment of a probe in the form of a urinary catheter;
Fig. 2 is a sideview of an embodiment of a probe in the form of a rectal probe;
Fig. 3 is perspective view of an embodiment of a probe in the form of another type of rectal probe;
Fig. 4 is a schematic sideview of an embodiment of a probe with a biofuel cell and sensors;
Fig. 5 is a schematic sideview of another embodiment of a probe with a biofuel cell and sensors;
Fig. 6 is a schematic perspective view of a control unit with an integrated display in accordance with an embodiment;
Fig. 7 is a schematic illustration of a disposable medical device in accordance with a first embodiment;
Fig. 8 is a schematic illustration of a disposable medical device in accordance with a second embodiment;
Fig. 9 is a schematic illustration of a disposable medical device in accordance with a third embodiment; and
Figs. 10a-c are schematic illustrations of three alternative embodiments of biofuel cells.

### DETAILED DESCRIPTION OF CURRENTLY PREFERRED EMBODIMENTS

A disposable medical device assembly is provided, for measuring a physiological parameter in a urinary or gastrointestinal tract of a mammal. In particular, the medical device assembly is suitable for use on human users and patients.

The disposable medical device assembly comprises a probe for insertion into a bodily opening of the user/patient, such as into the urethra and/or the rectum.

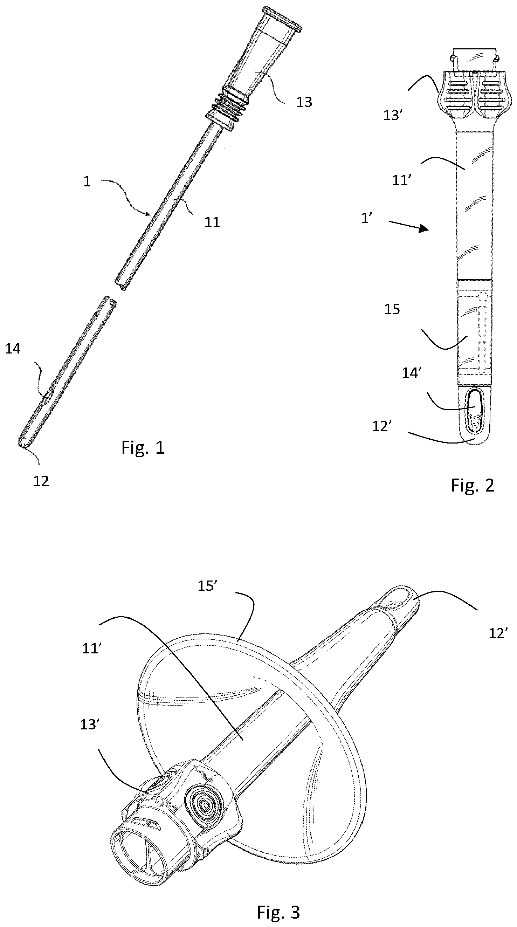

In one embodiment, as illustrated in Fig. 1, the probe is a urinary catheter 1. The catheter may comprise an elongate shaft 11, forming an insertable part of the catheter. At a non-insertable end 13 of the shaft, a connector, gripping portion or the like may be provided. At the other end of the shaft, a rounded insertable tip 12 may be formed, thereby forming a closed rounded end. The catheter is provided with an internal lumen. One or more drainage openings 14 may be provided close to the insertable tip 12. The internal lumen may extend over essentially the entire length of the shaft, from the drainage opening(s) 14 and to a discharge opening at the non-insertable end 13.

The probe/catheter may be provided with a coating on an outer surface of the shaft 11, e.g. to reduce the friction during insertion. The coating may e.g. be a hydrophilic coating. The hydrophilic coating can be wetted prior to use, e.g. with water or saline, and thereby swells and obtains lower friction.

In the embodiment of Fig. 1, a single internal lumen is provided. However, two or more lumens may also be provided. A single internal lumen may be used for a drainage catheter for draining urine from the bladder, and in particular for intermittent urinary catheters. An additional internal lumen may be used as a transfer channel for inserting a fluid into the urethra, such as a medicine. An additional internal lumen may also be use for an inflation fluid, for inflation and deflation of an inflatable retention element. This is particularly useful in case the catheter is intended as an indwelling catheter, for long time use, such as a Foley catheter. An additional lumen may also be used for housing a conducting wire connecting the sensor to the device platform.

As will be discussed in more detail in the following, the urinary catheter is provided with a biofuel cell and at least one sensor.

The probe may also be adapted for insertion into the rectum. The probe may e.g. be used for anal irrigation. Such an embodiment is shown in Fig. 2. Here, the probe 1' comprises an elongate shaft 11', forming an insertable part of the probe. At a non-insertable end 13' of the shaft, a connector, gripping portion or the like may be provided. At the other end of the shaft, a rounded insertable tip 12' may be formed, thereby forming a closed rounded end. The catheter is provided with an internal lumen. One or more discharge openings 14' may be provided close to the insertable tip 12', for discharge of an irrigation liquid, such as water. The internal lumen may extend over essentially the entire length of the shaft, from the discharge opening(s) 14' and to a discharge opening at the non-insertable end 13'.

This probe may also be provided with a coating on an outer surface of the shaft 11', e.g. to reduce the friction during insertion. The coating may e.g. be a hydrophilic coating. The hydrophilic coating can be wetted prior to use, e.g. with water or saline, and thereby swells and obtains lower friction.

Here, the probe is further provided with an inflatable retention element 15, an inflatable balloon, which may be expanded when the probe has been inserted, to maintain the probe in the inserted position. The retention element may be filled with air, liquid, or other fluids. To this end, a second internal lumen may be provided to communicate with the inflatable retention element. Such a probe is suitable for trans-anal irrigation (TAI).

In the embodiment of Fig. 2, two internal lumens are provided. Three or more lumens may also be provided. However, a probe with only one internal lumen is also feasible. In such embodiments, there may be no retention element, or a retention element which is not inflatable. Such an embodiment is illustrated in Fig. 3, where a retention element 15' in the form of a cone is provided. The cone is arranged to abut against the rectal opening, thereby forming a stop for too deep insertion. Such a probe is e.g. suitable for enema treatment.

As will be discussed in more detail in the following, the probes are provided with a biofuel cell and at least one sensor.

Alternatively, the probe may have no internal lumen. Such a probe may function as an anal plug or urethral plug, to form a stop in the urethra or rectum. Alternatively, such a probe may function only to provide measurements in the urinary or gastrointestinal tract. Such a probe may be formed as a solid probe, or be provided with internal cavities for improved flexibility. The flexibility may further be adjusted by choice of material, manufacturing processes, etc.

The probe may have an essentially circular cross-sectional shape. Such shapes are of particular advantage when the probe includes one or more internal lumens. However, other shapes are also feasible, such as an oval shape and the like. In other embodiments, in particular where there is no lumen inside the probe, the probe may also have a relatively flat cross-sectional shape, and may e.g. be formed as a rod, a strip, or the like.

The probe is preferably made of a relatively flexible material, making the probe somewhat bendable. In particular a probe for insertion into the urethra may have properties similar to a conventional urinary catheter. A probe for insertion into the rectum may have similar properties, but may alternatively be larger and somewhat more stiff and rigid, and generally have the properties of a rectal irrigation probe.

The medical device may comprise a control unit connected to or integrated with the probe. Such embodiments will be discussed in the following.

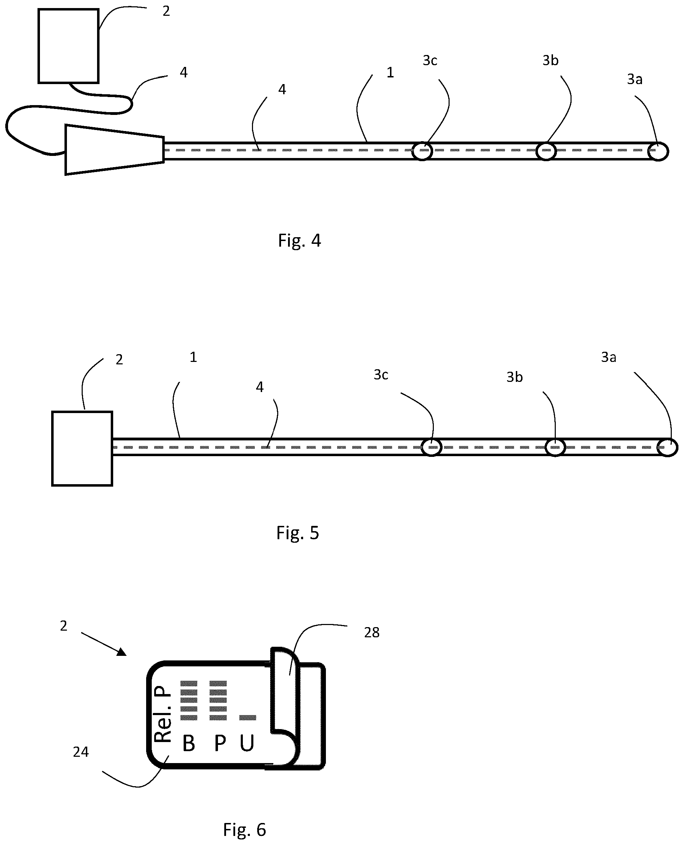

In one embodiment, illustrated in Fig. 4, a control unit 2 is arranged as a separate unit, connected to the probe 1 by a wire 4. The control unit is preferably arranged to be connected by the wire to the non-insertable rearward part of the probe. The probe is provided with sensors 3, as will be discussed in more detail in the following. In this embodiment, three sensors 3a-c are provided. The wire 4 connects the control unit 2 to the probe and continues in the probe to the sensors 3a-c. The wire may be arranged internally within the probe, arranged in an internal lumen of the probe or embedded in the material forming the probe. Alternatively, the wire may be arranged attached to an outer surface of the probe.

The wire may contain one or more conduits. The wire is arranged to convey signals representing measurement readings from the sensor(s) to the control unit. In case the sensors are electrically operated, the wire may further be arranged to supply electrical power to the sensors. The wire may e.g. be realized as a ribbon cable. However, other forms of wired connections are also useable to enable transfer of electricity from the biofuel cell and electrical signals or data signals from the sensors.

In another embodiment, illustrated in Fig. 5, the control unit 2 is instead formed as an integrated part of the probe, thereby forming a single unit. Also in this embodiment, the control unit is arranged at, or close to, the rearward, non-insertable part of the probe. However, in alternative embodiment, the control unit may also be provided at other positions on the probe, such as being integrated in the insertable part. As in the previously discussed embodiment, the sensors 3a-c of the probe are connected to the control unit 2 by a wire 4.

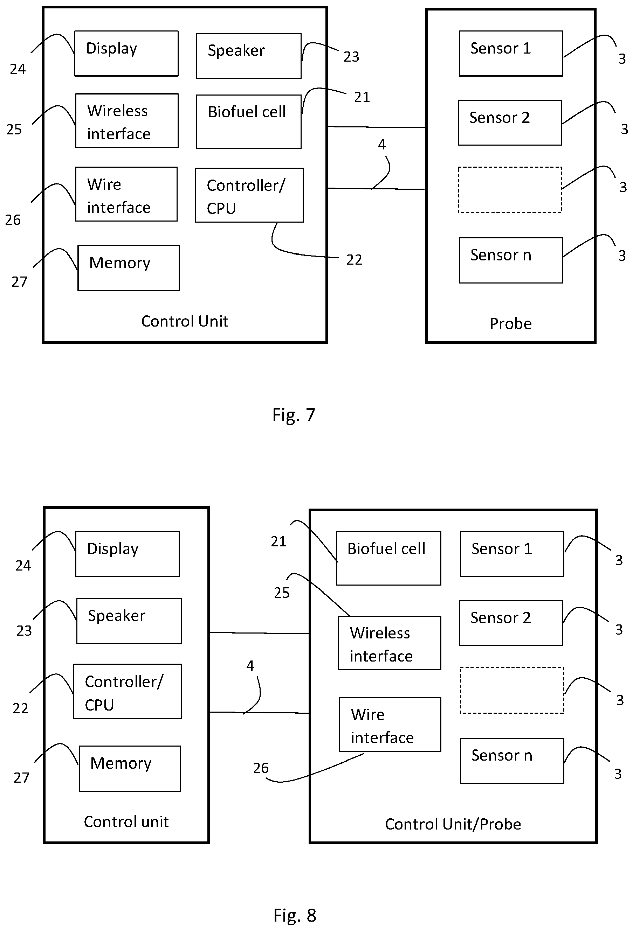

With reference to Fig. 7, the control unit comprises a biofuel cell 21, arranged to provide electrical power for operation of the control unit, and possibly also for the operation of the sensor(s) 3. The control unit may also comprise a controller 22, such as a central processing unit (CPU) or a microprocessor, arrange to receive measurement data from the one or more sensor 3, to determine useful information from this measurement data, and to present this in an adequate way to the user. To this end, the control unit may further comprise a user interface, such as a speaker 23, i.e. a sound generator, and/or a display 24. The speaker may also be a buzzer, or other types of sound generating devices. Further, the user interface may, additionally or alternatively, use haptic signals, such as vibrations. The speaker 23 may e.g. be used to issue an alarm when a predetermined event has happened, such as a threshold level being exceeded for a measured parameter. The display 24 may e.g. be used to present the measured data in the form of bars, diagrams, digits and the like. The control unit may, additionally or alternatively, comprise a communication interface for communication with an external unit, such as a personal computer, a tablet, a smartphone, or the like. To this end, the communication unit may comprise a wireless interface 25, comprising a wireless transmitter or transceiver for wireless communication with the external unit. Communication may e.g. occur through Bluetooth or NFC (Near Field Communication), but other communication protocols may also be used. Additionally, or alternatively, the control unit may also comprise a wire interface 26. Such an interface may comprise a connector, such as a plug or a jack, to establish a wired connection between the control unit and the external unit. The control unit may also comprise a memory 27, for storing of measured data. Such stored data may be used for the evaluation of subsequent measurements. The interpretation of the acquired data can be done in multiple ways, e.g. locally by the unit, by the external device where data was transferred, or by a cloud service that receives such data from either the unit or the external device where data was firstly transferred. Finally, the results of such interpretation could be displayed directly in the unit's UI or in a second device, e.g. a mobile app as user interface, a webpage as user interface, a computer application as user interface, an SMS sent containing results, or an email containing results. Those results could be accessed by the patient/user and/or the HCP.

As would be apparent from the previous discussion, all the discussed elements need not be present in the control unit. In a simple embodiment, the control unit may comprise only a biofuel cell and optionally a controller. In other embodiments, the control unit may in addition comprise either a user interface element, such as a speaker or display, or a communication interface, such as a wire or wireless interface.

The biofuel cell is preferably arranged to provide all the electrical power needed for the operation of all elements included in the control unit.

In the embodiment of Fig. 7, all the elements of the control unit are arranged in a separate part forming the control unit. The separate part may, as discussed in the foregoing, be arranged separated from the probe, and connected to the probe by the wire 4, or be arranged fixedly attached to the probe.

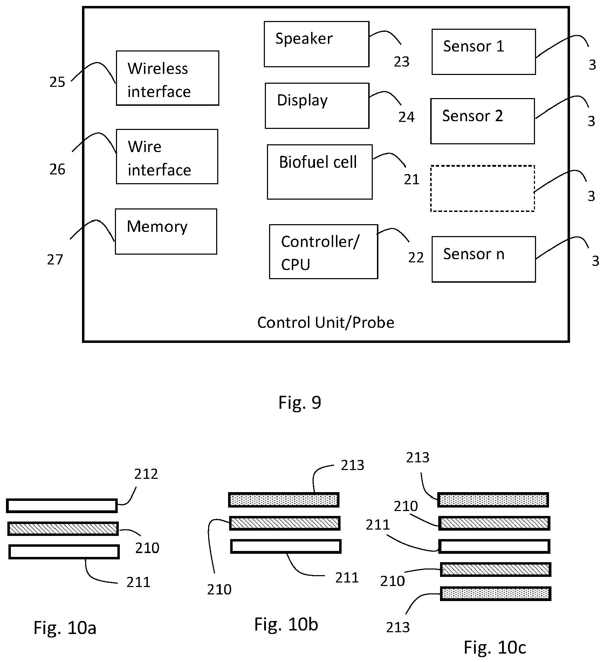

However, it is also possible to arrange one or more elements of the control unit integrated with the probe. It is also feasible to arrange one or more elements of the control unit integrated with the probe and one or more elements of the control unit in a separate control unit, to form a distributed control unit. Such embodiments are schematically illustrated in Figs. 8 and 9.

In the embodiment of Fig. 8, the biofuel cell 21 is arranged integrated with the probe, and also a communication interface, such as a wireless interface 25 and/or a wire interface 26. By a wired connection 4, or a wireless connection, this part of the control unit then communicates with another part of the control unit, comprising e.g. a controller 22 and a user interface, such as a speaker 23, buzzer, vibrator and/or a display 24, and optionally also a memory 27. This part of the control unit may be formed as a dedicated control unit, but may also be integrated in a smart phone, tablet, computer or the like.

In the embodiment of Fig. 9, all the elements of the control unit are arranged integrated with the probe.

The control unit may e.g. be arranged with an integrated display formed on one of its main surfaces, as illustrated in Fig. 6. The other elements of the control unit, such as the biofuel cell, may be arranged in other layers, to form a sandwiched construction. On the side of the control unit opposite to the display 24, an adhesive layer 28 may be arranged, to enable fastening of the control unit to an external surface, such as to the body of the user.

The biofuel cell 21 functions as the energy source for the disposable medical device, and preferably as the sole energy source. The biofuel cell may e.g. be made as discussed in any one of the published patent applications.

The biofuel cell is preferably free of metal, and preferably to a large extent made of biobased, compostable and/or biodegradable materials. In particular, the biofuel cell may be an enzymatic fuel cell, and preferably be paper-based. The biofuel cell may be arranged to use biological catalysts instead of chemical or expensive noble metal catalysts to convert natural substrates such as glucose and oxygen into electricity. The biofuel cell preferably uses biofuel enzyme cells to produce electrical energy from biological substances, such as glucose and oxygene, which are present in some biological fluids, such as blood sweat and urine.

The biofuel cell may be a single cathode cell (SC), as schematically illustrated in Fig. 10a, where a cathode 210 is positioned between an anode 211 and a support 212.

Alternatively, as schematically illustrated in Fig. 10b, the biofuel cell may be realized as a single cathode air cell (SABC). In a SABC realization, the support of the SC realization may be replaced with a support 213 which is permeable to air and allows penetration of oxygen. The support 213 may, as in the previous embodiment, be arranged above the cathode 210, and the anode 211 arranged below the cathode 210.

In yet another alternative, as schematically illustrated in Fig. 10c, the biofuel cell may be realized as a double cathode air cell (DABC). In the DABC realization, the biofuel cell comprises two cathodes 210 arranged on each side of an anode 211, and with permeable support layers 213 arranged on the outside of each of the two cathodes 210.

However, other realizations of the biofuel cell are also feasible, as is per se known in the art.

The anode(s) can e.g. be made of a solid agglomerate comprising a conductive material mixed with a first enzyme capable of catalyzing the oxidation of the biofuel. Similarly, the cathode(s) can e.g. be made of a solid agglomerate comprising a conductive material mixed with a second enzyme capable of catalyzing the reduction of oxidant.

The biofuel cell extracts electrons from oxidative reactions of biological fuels, using body fluid as an electrolyte solution and utilizing biological fuels, such as glucose, and oxygen from the body fluid. Any oxidative enzymes that oxidize biological fuels can be used in the present invention. For example, enzymes called oxidases and hydrogenases could be used. If glucose is used as the biological fuel, glucose oxidase and glucose dehydrogenase can be used.

The one or more sensor(s) is useable to determine one or more physiological parameters in relation to the user. The measured sensor data can be used to monitor physiological parameters, and thereto related health related conditions, over longer or shorter periods of time. The measured sensor data can also be used for diagnostic purposes, and also as an aid in various forms of treatments.

In case several sensors are provided on the probe, the sensors may be of the same type, such as several pressure sensors, e.g. arranged to measure the same physiological parameter at different positions. However, in alternative embodiments, sensors of different types may also be combined, to enable simultaneous measurement of two or more different physiological parameters.

The at least one sensor is preferably at least one of a pressure sensor, a temperature sensor and a flow sensor.

The sensor(s) may be arranged to determine one or more of the following physiological parameters: pressure, fluid flow rate, particle size, particle count, particle distribution, particle concentration, particle characteristics, analyte concentration, refractive index, conductivity, temperature, absorption spectrum, refraction spectrum, viscosity, and pH.

In one embodiment, the at least sensor comprises at least one pressure sensor, also referable to as a pressure transducer. The pressure sensor can e.g. be a digital or solid state pressure sensor, as is per se known in the art. Such pressure sensors may include, by way of example, any of a piezoelectric electric mechanism, an optical sensing mechanism, or a microelectricalmechanical (MEMS) mechanism.

In the embodiments of Figs. 4 and 5, three sensors 3a-c are provided, and separated along the length of the probe 1. The sensors 3a-c may e.g. be pressure sensors. When used in a probe arranged for insertion into the urethra, the sensor 3a may be arranged at, or in the vicinity of, the insertable tip. This sensor may then be used to determine the pressure inside the bladder. The other two sensors are arranged at a distance from the tip. The sensor 3b may here be arranged at a position which corresponds to the location of the prostate when the probe is in the inserted position, and consequently measures the pressure in the vicinity of the prostate. The third sensor 3c may be positioned at a distance from the prostate, and consequently measures the ordinary pressure of the urethra.

The display of Fig. 6 shows an example of how such a measurement may be presented. Here, the relative pressure ("Rel. P") is presented in the form of bars, and with one bar for each of the three positions: B = bladder, P = Prostate, and U = Urethra. From this, it is easily determinable whether the pressure differs to any significant degree between the three positions, during micturition, which could be an indication of urine obstruction.

Other types of sensors may also be used, in addition, or as alternatives, to the above-discussed pressure sensors, temperature sensors and flow sensors. Such sensors are per se known in the art.

Such other sensors may e.g. be analyte sensors, to determine, quantitatively or qualitatively, the presence of analytes such as pH, a gas, an electrolyte, a metabolic substrate, a metabolite, an enzyme, or a hormone. The analyte to be determined may also comprise one or more of proteins, glucose, hemoglobin, bilirubin, urobilinogen, acetone, nitrite and leucocytes. The analyte to be determined may further be fecal occult blood (FOB).

In some variations, at least one sensor may comprise one or more of a conductivity sensor, image sensor, accelerometer, and magnetic field transducer.

The sensors may also be arranged as electrical activity sensors. Such electrical activity sensors may deliver physiologic data such as electromyography (EMG) or electrogastrogram (EGG).

In some embodiments, the medical device further includes a light source and a light sensor, the sensor configured to capture light emitted from the light source. In some embodiments, by way of example, the light source and the light sensor may be configured to operate as a pulse oximeter, the light sensor being able to deliver a signal that can be transduced into a pulse rate. In another example, the light source and the light sensor may be configured to operate as an analyte sensor.

The sensor(s) may be arranged on an outer surface of the probe, and may e.g. be affixed to the probe by adhesive or the like. However, additionally or alternatively, sensor(s) may integrated in the probe, and/or be arranged inside the probe, such as in an internal lumen of the probe.

The disposable medical device may be packed in a sterile package. In use, the medical device, the package is opened, and the sterile probe is then extracted from the package, and inserted into the bodily opening, such as the urethra. The probe is then maintained in the inserted position during the measurement, which may be of a duration of a few seconds, a few minutes, or a few hours, depending on the measurement situation and the physiological parameter to be measured. After the measurement, the single-use device may be retracted and discarded.

The person skilled in the art realizes that the present invention by no means is limited to the preferred embodiments described above. On the contrary, many modifications and variations are possible within the scope of the appended claims. For example, various types of sensors and probes may be used.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

## Claims

1. A disposable medical device assembly for measuring a physiological parameter in a urinary or gastrointestinal tract of a mammal being, the assembly comprising:
a probe for insertion into a bodily opening of said mammal being, said probe comprising at least one sensor; and
an energy source connected to said at least one sensor;
wherein said energy source is a biofuel cell.

2. The disposable medical device assembly of claim 1, further comprising an interface arranged to transfer measurement data from said sensor to an external receiver.

3. The disposable medical device assembly of claim 2, wherein the interface comprises a connector arranged to connect to a wire.

4. The disposable medical device assembly of claim 2, wherein the interface is a wireless interface, and comprises a transmitter for wireless communication.

5. The disposable medical device assembly of claim 2, wherein the interface comprises at least one of a display and a speaker.

6. The disposable medical device assembly of any one of the preceding claims, wherein the probe is made of a biodegradable material.

7. The disposable medical device assembly of any one of the preceding claims, wherein the biofuel cell comprises a microbial and/or enzymatic solution, and wherein the biofuel cell preferably is an enzymatic fuel cell.

8. The disposable medical device assembly of any one of the preceding claims, wherein the biofuel cell is made of a biodegradable and/or compostable material, and preferably a metal free organic material .

9. The disposable medical device assembly of any one of the preceding claims, wherein the biofuel cell and/or the probe is made of a biobased material.

10. The disposable medical device assembly of any one of the preceding claims, wherein the at least one sensor is at least one of a pressure sensor, a temperature sensor, a position sensor, an imaging sensor, an accelerometer a gyroscope, a pH sensor and a flow sensor.

11. The disposable medical device assembly of any one of the preceding claims, wherein the at least sensor comprises at least one pressure sensor.

12. The disposable medical device assembly of claim 11, wherein the at least one sensor comprises a sensor arranged in the vicinity of an insertable tip of the probe, and at least one sensor arranged on the insertable part of the probe at a distance form said insertable tip.

13. The disposable medical device assembly of any one of the preceding claims, wherein the probe is arranged for insertion into the urethra or rectum of the mammal being.

14. The disposable medical device assembly of any one of the preceding claims, wherein the probe is arranged for insertion into the urethra of the mammal being, and comprising at least two sensors, one arranged in the vicinity of an insertable tip of the probe, for measurement of a pressure inside the bladder, and at least one sensor arranged on the insertable part of the probe at a distance form said insertable tip, for measurement of the pressure inside the urethra.

15. The disposable medical device assembly of claim 14, wherein the at least one sensor for measurement of the pressure inside the urethra comprises one sensor arranged to measure the pressure in the vicinity of the prostate, and one sensor arranged to measure the pressure inside the urethra at a distance from the prostate.

16. The disposable medical device assembly of any one of the preceding claims, further comprising a second probe, the second probe also comprising at least one sensor.

17. The disposable medical device assembly of any one of the preceding claims, wherein the biofuel cell is arranged at a distance from said at least one sensor, and wherein a conductor is provided in the probe forming a conductive path between the biofuel cell and the sensor(s).
